# EUROPÄISCHE PATENTANMELDUNG

(11) **EP 1 728 800 A1**
(43) Veröffentlichungstag der Anmeldung: **06.12.2006**
(21) Anmeldenummer: 06009351.5
(22) Anmeldetag: 09.10.1995
(51) Int. Cl.: C07K 14/025, C07K 19/00, C12N 15/12, A61K 39/12

(54) **Papillomavirusähnliche Partikel, Fusionsproteine sowie Verfahren zu deren Herstellung**

(30) Priorität: 07.10.1994 DE 4435907; 21.07.1995 DE 19526752
(62) Teilanmeldung aus: 95934663.6
(71) Anmelder: LOYOLA UNIVERSITY OF CHICAGO, Chicago, IL 60611 (US)
(72) Erfinder: Gissmann, Lutz, 69168 Wiesloch (DE); Zhou, Jian, deceased (US); Müller, Martin, 69151 Neckargemünd (DE); Painstil, Jeanette, Burr Ridge, Illinois 60527 (US)
(74) Vertreter: UEXKÜLL & STOLBERG

(57) **Zusammenfassung**

Die Erfindung betrifft rekombinant hergestellte papillomavirusähnliche Partikel, die nach Expression der viralen Strukturproteine L1 und/oder L2 entstehen, in denen ein oder mehrere Abschnitte des L1- und/oder L2-Proteins deletiert sind und wobei die Fähigkeit zur Bildung von virusähnlichen Partikeln im Vergleich zur nativen Bildung und/oder *in vitro* Produktion zumindest bestehen bleibt, bevorzugt erhöht ist.

SEQUENZPROTOKOLL

<110>  LOYOLA UNIVERSITY OF CHICAGO

<120>  PAPILLOMAVIRUSÄHNLICHE PARTIKEL, FUSIONSPROTEINE SOWIE VERFAHREN ZU
       DEREN HERSTELLUNG

<130>  P 70679

<140>  EP06009351.5
<141>  1995-10-09

<150>  DE19944435907
<151>  1994-10-07

<150>  DE19951026752
<151>  1995-07-21

<160>  4

<170>  PatentIn version 3.3

<210>  1
<211>  26
<212>  PRT
<213>  Papillomavirus sylvilagi

<400>  1

Gly Ala Gly Cys Ser Thr Val Arg Lys Arg Arg Ile Ser Gln Lys Thr
1               5                   10                  15

Ser Ser Lys Pro Ala Lys Lys Lys Lys Lys
            20                  25

<210>  2
<211>  78
<212>  DNA
<213>  Papillomavirus sylvilagi

<400>  2
ggggcaggat gttcaactgt gagaaaacga agaattagcc aaaaaacttc cagtaagcct      60

gcaaaaaaaa aaaaaaaa                                                    78

<210>  3
<211>  34
<212>  PRT
<213>  Papillomavirus sylvilagi

<400>  3

Ala Gly Leu Lys Ala Lys Pro Lys Phe Thr Leu Gly Lys Arg Lys Ala
1               5                   10                  15

Thr Pro Thr Thr Ser Ser Thr Ser Thr Thr Ala Lys Arg Lys Lys Arg
            20                  25                  30

Lys Leu

<210> 4
<211> 102
<212> DNA
<213> Papillomavirus sylvilagi

<400> 4
gcaggattga aggccaaacc aaaatttaca ttaggaaaac gaaaagctac acccaccacc 60

tcatctacct ctacaactgc taaacgcaaa aaacgtaagc tg 102

## Beschreibung

Die Erfindung betrifft rekombinant hergestellte papillomavirusähnliche Partikel, Proteine, Fusionsproteine sowie Verfahren zur Bildung und Reinigung dieser Partikel, Proteine und Fusionsproteine.

Infektionen mit bestimmten ("high-risk") Typen von genitalen Papillomaviren des Menschen (HPV), z.B. HPV 16, 18 oder 45 gelten als hauptsächlicher Risikofaktor für die Entstehung von bösartigen Tumoren des Anogenitaltrakts, von denen Gebärmutterhalskrebs (Cervixcarcinom) mit Abstand am häufigsten ist. Nach einer Schätzung der WHO treten jährlich weltweit etwa eine halbe Million neuer Fälle dieser Erkrankung auf. Aufgrund dieser Häufung ist der Zusammenhang zwischen HPV-Infektion und Cervixcarcinom am besten untersucht:
a) Vorläuferläsionen vom Cervixcarcinom (cervikale intraepitheliale Neoplasie: CIN) werden durch Papillomavirus-Infektion verursacht.
b) Die Genome bestimmter HPV-Typen (z.B. 16,18,33,35,45) werden in mehr als 95 % der Tumorbiopsien sowie in davon abgeleiteten Zellinien nachgewiesen. Abhängig vom geographischen Ursprung der Tumore enthalten 50 - 70 % davon HPV 1'6.
c) In allen daraufhin untersuchten Fällen werden die offenen Leseraster E6 und E7 transkribiert (Wettstein et al., in Pfister H. (ed): Papillomaviruses and human cancer, S. 155 bis 179, Boca Raton, 1990).
d) Die Proteine E6 und E7 sind in allen Cervixcarcinom-Zellinien sowie in *in vitro* transformierten menschlichen Keratinozyten nachweisbar und die Mehrzahl der Cervixcarcinom-Patientinnen haben E6- bzw. E7-spezifische Antikörper.
e) Die konstitutive Expression der E6/E7-Proteine ist notwendig zur Aufrechterhaltung des transformierten Zustandes HPV-positiver Tumore.
f) Die E6- und E7-Gene von HPV 16 und HPV 18 sind biologisch in folgenden experimentellen Systemen aktiv:
   - Induktion von zellulärer DNA Synthese in menschlichen Zellen;
   - Transformation von menschlichen Keratinozyten und anderen Zellen in Kultur;
   - Tumorbildung in transgenen Mäusen.

Andere HPV-Typen (in erster Linie HPV 6 und 11) verursachen gutartige Genitalwarzen (condylomata acuminata) und sind nur extrem selten mit bösartigen Tumoren assoziiert ("low-risk" Typen).

Genitale Papillomaviren des Menschen werden in der Regel durch Geschlechtsverkehr übertragen und führen in den meisten Fällen zu einer persistierenden Infektion in der Anogenital-Schleimhaut. Daraus wurde geschlossen, daß Primärinfektionen nur eine ungenügende Immunantwort induzieren oder daß das Virus Möglichkeiten entwickelt hat, in den infizierten Zellen der Immunüberwachung zu entkommen. Auf der anderen Seite gibt es gute Hinweise darauf, daß das Immunsystem bei der Primärmanifestation bzw. bei der malignen Progression von Papillomavirus-Infektionen beteiligt ist (zur Übersicht siehe Altmann et al. (1994) in Minson A., Neil J., McCrae M. (eds): Viruses and Cancer, Cambridge University Press, S. 71 bis 80).
a) Bei animalen Papillomaviren (Kaninchen-Papillomavirus und Rinder-Papillomavirus) läßt sich die klinische Manifestation von Primärinfektionen durch Vakzinierung mit Virus-Strukturproteinen oder mit Warzenextrakten ("autologe Vakzine") verhindern.
b) Nager werden durch Impfung mit HPV 16 E6- oder E7-positiven Vaccinia-Rekombinanten bzw. durch synthetische Peptide vor der Tumorbildung nach Inokulation HPV 16 transformierter autologer Zellen geschützt.
c) Regression von Warzen ist oftmals systemisch und läßt sich bei animalen Papillomaviren durch Transfer von Lymphozyten von "Regressor"-Tieren induzieren.
d) Die Häufigkeit von Genitalwarzen, CIN und Anogenitalkrebs ist bei immunsupprimierten Patienten (z.B. Nierentransplantierten oder HIV-Infizierten) erhöht.

Daraus wurde geschlossen, daß Papillomavirus-spezifische Impfungen mit dem Ziel der Verhinderung der Primärinfektion und der Entstehung von Genitalkrebs möglich sein sollten.
1. Geeignet ist die Verhinderung von HPV-Infektionen durch Impfung mit den Papillomavirus-Strukturproteinen L1 und L2 (prophylaktische Impfung).
   Da sich Papillomaviren nicht in Zellkultur oder anderen experimentellen Systemen zu ausreichenden Titern vermehren lassen, können die viralen Proteine nur mit Hilfe rekombinanter Vektoren hergestellt werden. Kürzlich wurden virusähnliche Partikel (VLP), die nach Expression der viralen Strukturproteine L1 und L2 (bzw. L1 allein) in rekombinanten Vakzinia oder Baculovirus entstehen, beschrieben. Die Reinigung der VLP's ist sehr einfach mittels Zentrifugation in CsCl- oder Sucrosegradienten durchführbar.
   WO 93/02184 beschreibt eine Methode, die "Papilloma virus like particles" (VLP's, Papillomavirus-ähnliche Partikel) zur Verfügung stellt, die für diagnostische Verwendungen oder als Vaccine gegen durch den Papillomavirus verursachte Infektionen genutzt werden.
   WO 94/00152 beschreibt ein rekombinant produziertes L1-Haupt-Capsid-Protein, welches die konformational neutralisierenden Epitope auf humanen und tierischen Papilloma-Virionen nachahmt (mimics). Diese rekombinanten Proteine sind als Vaccine, die gegen Papillomavirus-Infektionen schützen, einsetzbar.
2. Behandlung von Cervixcarcinomen oder Vorläuferläsionen durch Immuntherapie mit Hilfe von frühen Papillomavirus-Proteinen (in erster Linie E6 bzw. E7), die in den persistent infizierten Zellen exprimiert werden (therapeutische Impfung).
   Es wird angenommen, daß durch diese Impfung zytotoxische T-Zellen gegen persistent infizierte Genitalläsionen aktiviert werden. Zielpopulation sind Patienten mit HPV-assoziierten prämalignen oder malignen Genitalläsionen.
   Frühe HPV-Proteine werden durch Expression in E. coli oder eukaryontischen Vektoren (z.B. Baculovirus oder Hefe) hergestellt. Die Reinigung wird jedoch durch die geringe Löslichkeit erschwert und bedarf in der Regel einer Kombination von Ionenaustausch-Chromatographie, Gelfiltration und Affinitätschromatographie.
   In der PCT-Anmeldung WO 93/20844 wird dargelegt, daß das E7-Protein des Papillomavirus aus HPV oder BPV therapeutisch effektiv in der Regression (jedoch nicht in der Prävention) von Papillomavirus-Tumoren in Säugern ist. Weiterhin werden auch bevorzugte antigene Protein-Fragment-Sequenzen beschrieben.
   Bisher wurden jedoch keine VPL's beschrieben, die sich sowohl für die prophylaktische als auch die therapeutische Impfung eignen. Nachteilig ist bei den zuletzt genannten Verfahren, daß z.B. frühe HPV-Proteine aufgrund ihrer geringen Löslichkeit nur erschwert gereinigt werden können.
   Besonders wünschenswert, insbesondere im Hinblick auf einen Impfstoff für die prophylaktische und therapeutische Impfung wäre eine hohe Partikelproduktion.
   Nachteilig war an dem bisher beschriebenen Verfahren, daß die Herstellung von VLPs nach Expression von L1 in E. coli nicht möglich war.
   Die Aufgabe der vorliegenden Erfindung ist es daher, rekombinant hergestellte Proteine sowie VLP's zur Verfügung zu stellen, die sich als Impfstoff zur prophylaktischen und therapeutischen Impfung eignen, sowie Verfahren zur Herstellung dieser Proteine und VLP's. Ebenso soll eine einfache Reinigung der erhaltenen rekombinanten Proteine ermöglicht sein. Auch sollte eine Herstellung von VLPs nach Expression von L1 in E. coli ermöglicht sein.
   Die vorliegende Erfindung löst diese Aufgabe gemäß den in den unabhängigen Ansprüchen 1 und 12 angegebenen VLPs, den im unabhängigen Anspruch 36 angegebenen Proteinen, den in den unabhängigen Ansprüchen 8 und 38 angegebenen Fusionsproteinen, den in den Ansprüchen 42 und 43 angegebenen Verfahren, und der Verwendung nach den Ansprüchen 55 und 56. Weitere bevorzugte Ausgestaltungen, Aspekte und Details der' Erfindung sind in den abhängigen Patentansprüchen der Beschreibung sowie den bevorzugten Ausführungsformen dargelegt.
   Gemäß der vorliegenden Erfindung werden VLP's hergestellt, die aus Fusionsproteinen von späten und frühen HPV-Proteinen (oder Fragmenten davon) ("HVLP") bestehen und für prophylaktische bzw. therapeutische Impfung einsetzbar sind. Ein solcher Impfstoff besitzt gegenüber konventionellen Präparaten die im folgenden beschriebenen Vorteile:
   a) Im Falle von prophylaktischer Impfung verhindern HVLP's durch Induktion L1/L2-spezifischer Antikörper nicht nur den Eintritt des Virus in die Zelle, sondern eliminieren bereits infizierte Zellen (durch Induktion von zytotoxischen T-Zellen), falls schon früher eine Infektion stattgefunden hat oder die humorale Immunantwort nicht ausreichend war.
   b) Im Falle von therapeutischer Impfung eliminieren HVLP's persistent infizierte Zellen (z.B. bei Patienten mit CIN oder Cervixcarcinom), und verhindern vor allem bei Patientinnen mit CIN-Läsionen eine Reinfektion.
   c) Die Reinigung der HVLP's ist ähnlich einfach wie die der VLP's ohne frühe HPV-Proteine.

Gemäß der vorliegenden Erfindung können VLP's des Bovinen Papillomavirus (BPV) Typ 1 und der humanen Papillomaviren 11 und 16 nach Expression von L1 plus L2 bzw. von L1 allein in Vaccinia oder Baculovirus hergestellt werden. Experimente zeigen, daß Teile des L1-Proteins deletiert werden können (Aminosäuresequenz 311-351, 331-371, 391-431 von BPV 1; 306-315 von HPV 16), ohne daß die Fähigkeit zur Bildung von VLP's verloren geht. Solche Bereiche existieren in den L1-Proteinen aller Papillomaviren, so daß der deletierte Bereich von L1 durch andere Proteine (von Päpillomaviren oder von anderem Ursprung) ersetzt werden kann und daß so virusähnliche Hybridpartikel hergestellt werden können. In gleicher Weise werden auch Teile des Papillomavirus-Proteins L2 deletiert und durch andere (frühe HPV oder sonstige) Proteine ersetzt, daß also HVLP's auch aus dem vollständigen L1-Protein plus einem L2-Fusionsprotein gebildet werden können.

Fusionsproteine, bestehend aus deletiertem L1- oder L2-Protein von verschiedenen HPV-Typen (in erster Linie HPV 6, 11, 16, 18, 33, 35, 45) und den entsprechenden frühen Proteinen E1, E2, E4, E5, E6, E7 (oder Teilen davon) werden durch Expression in Vaccinia-Rekombinanten hergestellt, die in sehr kurzer Zeit konstruiert werden können. Die Bildung von VLP's, bestehend entweder aus einem L1-Fusionsprotein oder aus dem vollständigen L1-Protein plus einem L2-Fusionsprotein, wird durch Elektronenmikroskopie überprüft und das Vorhandensein des frühen HPV-Proteins durch Western Blot Analyse mit Hilfe spezifischer Antiseren getestet. Für die Produktion von HPLV's im großen Maßstab wird die Expression der Proteine in viralen oder eukaryotischen Systemen, bevorzugt in Baculovirus oder in Hefe, durchgeführt.

Entsprechende Experimente zur Herstellung von Fusionsproteinen können mit Proteinen anderen Ursprungs durchgeführt werden.

Wesentlich für die vorliegende Erfindung sind rekombinant hergestellte, virusähnliche Partikel (virus like particles, VLP's), die nach Expression der viralen Strukturproteine L1 und/oder L2 entstehen, wobei Abschnitte des L1- und/oder L2-Proteins deletiert sind, ohne daß die Fähigkeit zur Bildung von VLP's verlorengeht.

Gemäß der vorliegenden Erfindung handelt es sich bei dem deletierten Bereich im L1-Protein des Bovinen Papillomavirus Typ 1 bevorzugt um die Aminosäuresequenzen 311-351, 331-371, 391-431. Bei L1-Proteinen des humanen Papillomavirus 16 handelt es sich vorteilhafterweise um die Aminosäuresequenz 306-315.

In einer bevorzugten Ausgestaltung der vorliegenden Erfindung wird der deletierte Bereich von L1- und/oder L2-Proteinen durch andere Proteine oder Proteinfragmente ersetzt, wobei Fusionsproteine erhalten werden. Der Anteil an L1- bzw. L2-Protein beträgt vorteilhafterweise ca. 50 bis 99 %, bevorzugt ca. 60 bis 90 %, besonders bevorzugt ca. 80 %.

Gemäß der vorliegenden Erfindung sollen jedoch, wenn auch im weiteren nicht explizit erwähnt, auch mehr als ein Bereich, des L1- und/oder L2-Proteins deletiert und bevorzugt durch andere Proteine oder Proteinfragmente ersetzt werden.

Besonders bevorzugt wird der deletierte Bereich im L1- oder L2-Protein durch andere Proteine von Papillomaviren und/oder Proteine anderen Ursprungs ersetzt, wodurch virusähnliche Hybridpartikel (HVLP's) herstellbar sind.

Es hat sich als besonders vorteilhaft gemäß der vorliegenden Erfindung erwiesen, daß die Bildung der VLP's aus einem L1-Fusionsprotein oder, gemäß einer weiteren Ausführungsform, aus einem vollständigen L1-Protein und einem L2-Fusionsprotein erfolgt.

Die Fusionsproteine, insbesondere zur Bildung von virusählichen Hybridpartikeln gemäß einer weiteren Ausgestaltung der vorliegenden Erfindung, bestehen vorteilhafterweise aus einem deletierten L1- und/oder L2-Protein von verschiedenen HPV-Typen (human papilloma virus), besonders bevorzugt HPV 6, 11, 16, 18, 33, 35 und 45, und anderen Proteinen oder Proteinfragmenten. Bevorzugt handelt es sich bei diesen anderen Proteinen oder Proteinfragmenten um entsprechende frühe Proteine oder Fragmente davon, wie z.B. die frühen Proteine E1, E2, E4, E5, E6 und/oder E7.

Gemäß dem erfindungsgemäßen Verfahren wird die Expression der Fusionsproteine und Proteine in viralen oder eukaryotischen Vektoren, ganz besonders bevorzugt in Baculoviren oder in Hefen, durchgeführt.

Gemäß einer weiteren Ausgestaltung des erfindungsgemäßen Verfahrens werden die Fusionsproteine durch Expression in Vaccinia-Rekombinanten hergestellt.

Die Anwendung der Fusionsproteine oder der virusähnlichen Hybridpartikel zur Herstellung eines prophylaktischen und therapeutischen Impfstoffes erfolgt gemäß der vorliegenden Erfindung bevorzugt nach Zugabe weiterer Komponenten.

Bislang wurde zur Herstellung von VLPs, wie z.B. von VLPs aus HPV 16, das L1 offene Leseraster (ORF) mit Hilfe eukaryontischer Vektoren, wie z.B. Bakulovirus, exprimiert. Die Bildung der VLPs (Assembly) erfolgt im Zellkern der infizierten Zellen.

Wesentlich für die vorliegende Erfindung sind deshalb insbesondere rekombinant hergestellte, papillomavirusähnliche Partikel, die nach Expression der viralen Strukturproteine L1 und/oder L2 entstehen, in denen ein oder mehrere Abschnitte des L1- und/oder L2-Proteins deletiert sind, wobei die Fähigkeit zur Bildung von virusähnlichen Partikeln im Vergleich zur nativen Bildung und/oder *in vitro* Produktion erhöht ist.

Gemäß der vorliegenden Erfindung handelt es sich bei mindestens einem der deletierten Bereiche im L1- und/oder L2-Protein eines Papillomavirus um eine Deletion, vorteilhafterweise in der C-terminalen Aminosäuresequenz, bevorzugt in einer Länge von ungefähr 1 bis 34 Aminosäuren (AS), bevorzugt von 1 bis 26 Aminosäuren (AS), insbesondere von 26 AS.

Vorteilhafterweise wird nach Einfügen der C-terminalen Deletion in das L1- und/oder L2-Protein die Produktion von VLPs um ein Vielfaches erhöht, bevorzugt um das mindestens 10-fache, und insbesondere um das ungefähr 10- bis 100-fache.

In einer bevorzugten Ausgestaltung der vorliegenden Erfindung handelt es sich bei den deletierten Bereichen im L1- und/oder L2-Protein, insbesondere des Bovinen Papillomavirus, um 26C-terminale Aminosäuren. Besonders bevorzugt ist die 26 AS große, C-terminale Deletion (Gly-Ala-Gly-Cys-Ser-Thr-Val-Arg-Lys-Arg-Arg-Ile-Ser-Gln-Lys-Thr-Ser-Ser-Lys-Pro-Ala-Lys-Lys-Lys-Lys-Lys) entsprechend der Nucleotid-Position 7016 bis 7093 GGGGCAGGAT GTTCAACTGT GAGAAAACGA AGAATTAGCC AAAAAACTTC CAGTAAGCCT GCAAAAAAAA AAAAAAAA in das L1 ORF des Bovinen Papillomavirus Typ 1 (BPV 1) eingefügt. Vorteilhafterweise ist nach Einfügen der C-terminalen Deletion in das L1-und/oder L2-Protein die Produktion von VLP' s um das mindestens 10-fache gesteigert.

Gemäß einer weiteren Ausgestaltung der vorliegenden Erfindung handelt es sich bei der mindestens einen Deletion im L1-und/oder L2-Protein um eine homologe Aminosäuresequenz des humanen Papillomavirus 16 oder anderer Papillomaviren.

Gemäß einer weiteren bevorzugten Ausgestaltung handelt es sich bei den deletierten Bereichen im L1- und/oder L2-Protein um 34 C-terminale Aminosäuren des Humanen Papillomavirus Typ 16 (HPV 16), bevorzugt um die AS-Sequenz Ala-Gly-Leu-Lys-Ala-Lys-Pro-Lys-Phe-Thr-Leu-Gly-Lys-Arg-Lys-Ala-Thr-Pro-Thr-Thr-Ser-Ser-Thr-Ser-Thr-Thr-Ala-Lys-Arg-Lys-Lys-Arg-Lys-Leu) entsprechend der Nucleotid-Position 7052 bis 7153 GCAGGATTGA AGGCCAAACC AAAATTTACA TTAGGAAAAC GAAAAGCTAC ACCCACCACC TCATCTACCT CTACAACTGC TAAACGCAAA AAACGTAAGC TG, welche in das L1 ORF des HPV 16 eingefügt ist.

Besonders bevorzugt umfaßt die Deletion des L1- und/oder L2-Proteins das nukleäre Lokalisationssignal (NLS). Die Partikelproduktion aus den L1-Proteinen oder den L1-Proteinen und L2-Proteinen erfolgt insbesondere im Zytoplasma. Bevorzugt werden die Partikel in den Überstand sezerniert, besonders bevorzugt ist eine Sekretion von ungefähr 5 bis 10 % der Partikel.

Die Expression von L1-Proteinen oder L1-Proteinen und L2-Proteinen in E. coli erfolgt gemäß einer weiteren bevorzugten Ausführungsform. Hierbei sind an der C-terminalen Deletion im L1-Protein insbesondere zusätzlich 6 Histidine eingefügt. Vorteilhafterweise erfolgt die Herstellung von VLP's nach Expression von L1-Proteinen oder L1- und L2-Proteinen in E. coli.

Gemäß der vorliegenden Erfindung handelt es sich bei den weiteren deletierten Bereichen im L1-Protein des Bovinen Papillomavirus Typ 1 bevorzugt um die Aminosäuresequenzen 311-351, 331-371, 391-431. Bei L1-Proteinen des humanen Papillomavirus 16 handelt es sich vorteilhafterweise um die Aminosäuresequenz 306-315.

In einer bevorzugten Ausgestaltung der vorliegenden Erfindung wird der weitere deletierte Bereich von L1- und/oder L2-Proteinen durch andere Proteine oder Proteinfragmente ersetzt, wobei Proteine erhalten werden, die im weiteren als Fusionsproteine bezeichnet werden. Der Anteil an L1- bzw. L2-Protein beträgt vorteilhafterweise ca. 50 bis 99 %, bevorzugt ca. 60 bis 90 %, besonders bevorzugt ca. 80 %.

Gemäß der vorliegenden Erfindung sollen jedoch, wenn auch im weiteren nicht explizit erwähnt, auch mehr als ein weiterer Bereich des L1- und/oder L2-Proteins deletiert und bevorzugt durch andere Proteine oder Proteinfragmente ersetzt werden.

Besonders bevorzugt wird der deletierte Bereich von L1- oder L2-Protein durch andere Proteine von Papillomaviren und/oder Proteine anderen Ursprungs ersetzt, wodurch virusähnliche Hybridpartikel (HVLP's) herstellbar sind.

Es hat sich als besonders vorteilhaft gemäß der vorliegenden Erfindung erwiesen, daß die Bildung der VLP's aus einem L1-Protein, einem L1-Fusionsprotein, einem L1-Protein und L2-Protein, einem L1-Fusionsprotein und L2-Protein, einem L1-Protein und einem L2-Fusionsprotein oder einem L1-Fusionsprotein und einem L2-Fusionsprotein erfolgt.

Gemäß einer weiteren Ausgestaltung der vorliegenden Erfindung handelt es sich bei mindestens einem der deletieren Bereiche im L1- und/oder L2-Protein eines Papillomavirus um N-terminale Aminosäuresequenzen.

Gemäß der vorliegenden Erfindung handelt es sich bei einer weiteren Ausführungsform bei mindestens einem der deletierten Bereiche im L1-Protein und/oder L2-Protein eines Papillomavirus um Aminosäuresequenzen im mittleren Bereich des Proteins.

Wesentlich für die Erfindung sind ebenfalls Proteine, insbesondere zur Bildung von papillomavirusähnlichen Hybridpartikeln, wobei ein oder mehrere Abschnitte des L1-und/oder L2-Proteins deletiert sind. Insbesondere handelt es sich bei mindestens einem der deletierten Bereiche im L1-und/oder L2-Protein um eine Deletion einer C-terminalen Aminosäuresequenz.

Die Fusionsproteine, insbesondere zur Bildung von papillomavirusähnlichen Hybridpartikeln gemäß einer weiteren Ausgestaltung der vorliegenden Erfindung, bestehen vorteilhafterweise aus einem deletierten L1- und/oder L2-Protein von verschiedenen Papillomaviren, besonders bevorzugt HPV 6, 11, 16, 18, 31, 33, 35 und 45, und anderen Proteinen oder Proteinfragmenten von Papillomaviren oder von anderer Herkunft. Bevorzugt handelt es sich bei diesen anderen Proteinen oder Proteinfragmenten um entsprechende frühe Papillomavirus-Proteine oder Fragmente davon, wie z.B. die frühen Proteine E1, E2, E4, E5, E6 und/oder E7.

Gemäß dem erfindungsgemäßen Verfahren wird zur Expression der Proteine und/oder Fusionsproteine und Produktion von papillomavirusähnlichen Partikeln in viralen, eukaryotischen oder prokaryotischen Vektoren, ganz besonders bevorzugt in Vaccinia-Rekombinanten, in Baculoviren, in Hefen oder in Bakterien, insbesondere in E. coli, durchgeführt.

Bevorzugt erfolgt die Partikelproduktion im Zytoplasma. Die Partikel werden besonders bevorzugt in den Überstand sezerniert, ganz besonders bevorzugt werden ungefähr 5 bis 10 % der Partikel in den Überstand sezerniert.

Insbesondere wird gemäß der vorliegenden Erfindung durch Einfügen einer 26 Aminosäure (AS) großen, C-terminalen Deletion in der Nucleotidposition 7016-7093 in das L1 ORF des bovinen Papillomavirus Typ 1 (BPV 1) die Produktion von VLPs um das mehr als 10-fache gesteigert. So läßt sich bei gleicher Menge an L1-Protein, wie z.B. in einem Western Blot demonstrierbar, eine Steigerung der Partikelzahl im Elektronenmikroskop zeigen. Da die Deletion bevorzugt das nukleäre Lokalisationssignal (NLS) umfaßt, erfolgt die Partikelproduktion im Zytoplasma, ein erheblicher Teil der Partikel wird in den Überstand sezerniert. Dies ist besonders vorteilhaft, da hierdurch die Reinigung wesentlich erleichtert wird.

Proteine, bevorzugt mit genannter Deletion mit zusätzlichen 6 Histidinen (His-L1-Proteine) werden gemäß der vorliegenden Erfindung in E. coli exprimiert. Die Proteine, insbesondere His-L1-Proteine, werden bevorzugt über Ni-Affinitätschromatograpie gereinigt, wobei die Proteine entsprechend einer vorteilhaften Ausführungsform zu diesem Zeitpunkt in Denaturierungspuffer, beispielsweise 6 M Guanidinhydrochlorid, vorliegen. Die Renaturierung erfolgt beispielsweise in 150 mM NaCl, 1 mM CaCl₂, 0,01 % Triton-X 100, 10 mM Hepes (N-2-hydroxyethylpiperazine-N'-2-ethansulfonsäure), pH 7,4.

Die Produktion (Assembly) der VLP erfolgt gemäß einer bevorzugten Ausgestaltung der vorliegenden Erfindung nach Dialyse der Proteine, vorteilhafterweise nach Dialyse gegen 150 mM NaCl, 25 mM Ca²⁺, 10 % DMSO (Dimethylsulfoxid), 0,1 % Triton-X 100, 10 mM Tris [tris-(hydroxymethyl)aminomethan] Essigsäure bei einem pH-Wert von 5,0.

Die Deletion von Sequenzen im L1-Protein von sämtlichen Papillomaviren, die das vorzeitige Assembly der VLPs verhindern, führt zu einer höheren Ausbeute bei der VLP Produktion.

Sofern in diesen Fällen das L1 NLS betroffen ist, erfolgt die Assembly im Zytoplasma. Somit ist die Reinigung des VLPs erfindungsgemäß aus dem Zytoplasma, statt wie bisher aus dem Zellkern, möglich. Erfindungsgemäß sind auch kürzere Deletionen möglich. Gemäß der vorliegenden Erfindung werden Deletionen bis zu einer Aminosäure und/oder Substitutionen von bis zu einer Aminosäure durchgeführt. Hierbei erweist es sich als vorteilhaft, daß bei kurzen Deletionen bzw. bei Substitutionen von bis zu einer bzw. nur weniger Aminosäuren die antigenen Eigenschaften der Proteine und der sich daraus gebildeten VLPs so wenig wie möglich gegenüber der nativen antigenen Eigenschaft der Proteine bzw. VLPs verändert sind.

Die Einführung einer wie vorgehend ausgeführten C-terminalen Deletion bzw. Substitution in L1- und/oder L2-Fusionsproteine, führt auch zu einer Steigerung der Produktion von Hybrid VLPs. Hierbei sollen auch die VLPs eingeschlossen sein, die nur L1-Fusionsproteine enthalten, sowie Hybrid VLPs, die ein L1- bzw. L2-Fusionsprotein und ein L2- bzw. L1-Protein enthalten.

Hierzu werden VLP's hergestellt, die aus Fusionsproteinen von späten und frühen HPV-Proteinen (oder Fragmenten davon) ("HVLP") bestehen und für prophylaktische bzw. therapeutische Impfung einsetzbar sind. Ein solcher Impfstoff besitzt gegenüber konventionellen Präparaten die im folgenden beschriebenen Vorteile:
a) Im Falle von prophylaktischer Impfung verhindern HVLP's durch Induktion L1/L2-spezifischer Antikörper nicht nur den Eintritt des Virus in die Zelle, sondern eliminieren bereits infizierte Zellen (durch Induktion von zytotoxischen T-Zellen), falls schon früher eine Infektion stattgefunden hat oder die humorale Immunantwort nicht ausreichend war.
b) Im Falle von therapeutischer Impfung eliminieren HVLP's persistent infizierte Zellen (z.B. bei Patienten mit CIN oder Cervixcarcinom), und verhindern vor allem bei Patientinnen mit CIN-Läsionen eine Reinfektion.
c) Die Reinigung der HVLP's ist ähnlich einfach wie die der VLP's ohne frühe HPV-Proteine.

VLP's des Bovinen Papillomavirus (BPV) Typ 1 und der humanen Papillomaviren 11 und 16 können nach Expression von L1 plus L2 bzw. von L1 allein in Vaccinia oder Baculovirus hergestellt werden. Experimente zeigen, daß Teile des L1-Proteins deletiert werden können (Aminosäuresequenz 311-351, 331-371, 391-431 von BPV 1; 306-315 von HPV 16), ohne daß die Fähigkeit zur Bildung von VLP's verloren geht. Solche Bereiche existieren in den L1-Proteinen aller Papillomaviren, so daß der deletierte Bereich von L1 durch andere Proteine (von Papillomaviren oder von anderem Ursprung) ersetzt werden kann und daß so virusähnliche Hybridpartikel hergestellt werden können. In gleicher Weise werden auch Teile des Papillomavirus-Proteins L2 deletiert und durch andere (frühe HPV oder sonstige) Proteine ersetzt, daß also HVLP's auch aus dem vollständigen L1-Protein plus einem L2-Fusionsprotein gebildet werden können.

Fusionsproteine bestehend aus deletiertem L1- oder L2-Protein von verschiedenen HPV-Typen (in erster Linie HPV 6, 11, 16, 18, 33, 35, 45) und den entsprechenden frühen Proteinen E1, E2, E4, E5, E6, E7 (oder Teilen davon) werden durch Expression in Vaccinia-Rekombinanten hergestellt, die in sehr kurzer Zeit konstruiert werden können. Die Bildung von VLPs, bestehend entweder aus einem L1-Fusionsprotein oder aus dem vollständigen L1-Protein plus einem L2-Fusionsprotein, wird durch Elektronenmikroskopie überprüft und das Vorhandensein des frühen HPV-Proteins durch Western Blot Analyse mit Hilfe spezifischer Antiseren getestet. Für die Produktion von HPLV's im großen Maßstab wird die Expression der Proteine in viralen eukaryotischen oder prokaryotischen Systemen, bevorzugt in Baculovirus, in Hefe, oder in E. coli durchgeführt.

Die Anwendung der Fusionsproteine oder der virusähnlichen Hybridpartikel zur Herstellung eines prophylaktischen und therapeutischen Impfstoffs erfolgt gemäß der vorliegenden Erfindung bevorzugt nach Zugabe weiterer Komponenten.

Entsprechende Experimente zur Herstellung von Fusionsproteinen können mit Proteinen anderen Ursprungs durchgeführt werden.

### Annex zu den Anmeldungsunterlagen - nachgereichtes Sequenzprotokoll

## Patentansprüche

1. Rekombinant hergestellte papillomavirusähnliche Partikel, die nach Expression der viralen Strukturproteine L1 und/oder L2 entstehen, **dadurch gekennzeichnet, daß** ein oder mehrere Abschnitte des L1- und/oder L2-Proteins deletiert sind, wobei die Fähigkeit zur Bildung von virusähnlichen Partikeln bestehen bleibt.

2. Virusähnliche Partikel nach Anspruch 1, **dadurch gekennzeichnet, daß** es sich bei den deletierten Bereichen im L1-Protein um die Aminosäuresequenzen 311-351, 331-371, 391-431 des Bovinen Papillomavirus Typ 1 handelt.

3. Virusähnliche Partikel nach Anspruch 1, **dadurch gekennzeichnet, daß** es sich bei den deletierten Bereichen im L1-Protein um die Aminosäuresequenz 306-315 des humanen Papillomavirus 16 handelt.

4. Virusähnliche Partikel nach einem der vorhergehenden Ansprüche, **dadurch gekennzeichnet, daß** der deletierte Bereich von L1- und/oder L2-Proteinen durch andere Proteine oder Proteinfragmente ersetzt ist, um ein Fusionsprotein zu erhalten.

5. Virusähnliche Partikel nach Anspruch 4, **dadurch gekennzeichnet, daß** der Anteil an L1- bzw. L2-Protein ca. 50 bis 99 %, bevorzugt ca. 60 bis 90 %, besonders bevorzugt ca. 80 % des Fusionsproteins beträgt.

6. Virusähnliche Partikel nach Anspruch 4 und 5, **dadurch gekennzeichnet, daß** der deletierte Bereich im L1-oder L2-Protein durch andere Proteine von Papillomaviren und/oder Proteinen anderen Ursprungs ersetzt ist, wodurch virusähnliche Hybridpartikel herstellbar sind.

7. Virusähnliche Partikel nach einem der vorhergehenden Ansprüche, **dadurch gekennzeichnet, daß** die Bildung der virusähnlichen Partikel aus einem L1-Fusionsprotein oder einem vollständigen L1-Protein und einem L2-Fusionsprotein erfolgt.

8. Fusionsproteine, insbesondere zur Bildung von papillomavirusähnlichen Hybridpartikeln nach einem der Ansprüche 4 bis 7, **dadurch gekennzeichnet, daß** die Fusionsproteine aus einem deletierten L1-und/oder L2-Protein von verschiedenen HPV-Typen und andere Proteine oder Fragmente davon umfassen.

9. Fusionsprotein nach Anspruch 8, **dadurch gekennzeichnet, daß** die anderen Proteine oder Fragmente frühe Proteine oder Fragmente sind.

10. Fusionsprotein nach Anspruch 8 und 9, **dadurch gekennzeichnet, daß** es sich um die frühen Proteine E1, E2, E4, E5, E6, E7 oder um Fragmente davon handelt.

11. Fusionsprotein nach einem der Ansprüche 8 bis 10, **dadurch gekennzeichnet, daß** es sich um deletierte L1-und/oder L2-Proteine von verschiedenen HPV-Typen wie HPV 6, 11, 16, 18, 33, 35, 45 handelt.

12. Rekombinant hergestellte papillomavirusähnliche Partikel, die nach Expression der viralen Strukturproteine L1 und/oder L2 entstehen,
**dadurch gekennzeichnet,**
**daß** ein oder mehrere Abschnitte des L1- und/oder L2-Proteins deletiert sind, wobei die Fähigkeit zur Bildung von virusähnlichen Partikeln im Vergleich zur nativen Bildung und/oder in vitro Produktion erhöht ist.

13. Virusähnliche Partikel nach Anspruch 12, **dadurch gekennzeichnet, daß** es sich bei mindestens einem der deletierten Bereiche im L1- und/oder L2-Protein eines Papillomavirus um C-terminale Aminosäuresequenzen handelt.

14. Virusähnliche Partikel nach Anspruch 13, **dadurch gekennzeichnet, daß** es sich bei dem mindestens einen deletierten Abschnitt im C-terminalen Bereich im L1-und/oder L2-Protein um eine Aminosäuresequenz in einer Länge von ungefähr 1 bis 34 Aminosäuren, bevorzugt 1 bis 26 Aminosäuren, eines Papillomavirus handelt.

15. Virusähnliche Partikel nach einem der Ansprüche 12 bis 14, **dadurch gekennzeichnet, daß** nach Einfügen insbesondere der C-terminalen Deletion in das L1-und/oder L2-Protein die Produktion von VLPs um ein Vielfaches, bevorzugt um das mindestens 10-fache, besonders bevorzugt um das ca. 10- bis 100-fache, gesteigert wird.

16. Virusähnliche Partikel nach einem der Ansprüche 12 bis 15, **dadurch gekennzeichnet, daß** es sich bei den deletierten Bereichen im L1- und/oder L2-Protein um 26C-terminale Aminosäuren des Bovinen Papillomavirus handelt.

17. Virusähnliche Partikel nach Anspruch 16, **dadurch gekennzeichnet, daß** die 26 AS große, C-terminale Deletion (Gly-Ala-Gly-Cys-Ser-Thr-Val-Arg-Lys-Arg-Arg-Ile-Ser-Gln-Lys-Thr-Ser-Ser-Lys-Pro-Ala-Lys-Lys-Lys-Lys-Lys) entsprechend der Nucleotid-Position 7016 bis 7093 GGGGCAGGAT GTTCAACTGT GAGAAAACGA AGAATTAGCC AAAAAACTTC CAGTAAGCCT GCAAAAAAAA AAAAAAAA, in das L1 ORF des Bovinen Papillomavirus Typ 1 eingefügt ist.

18. Virusähnliche Partikel nach einem der Ansprüche 12 bis 15, **dadurch gekennzeichnet, daß** es sich bei den deletierten Bereichen im L1- und/oder L2-Protein um 34C-terminale Aminosäuren des humanen Papillomavirus Typ 16 (HPV 16) handelt.

19. Virusähnliche Partikel nach Anspruch 18, **dadurch gekennzeichnet, daß** die 34 AS große, C-terminale Deletion (Ala-Gly-Leu-Lys-Ala-Lys-Pro-Lys-Phe-Thr-Leu-Gly-Lys-Arg-Lys-Ala-Thr-Pro-Thr-Thr-Ser-Ser-Thr-Ser-Thr-Thr-Ala-Lys-Arg-Lys-Lys-Arg-Lys-Leu) entsprechend der Nucleotid-Position 7052 bis 7153 GCAGGATTGA AGGCCAAACC AAAATTTACA TTAGGAAAAC GAAAAGCTAC ACCCACCACC TCATCTACCT CTACAACTGC TAAACGCAAA AAACGTAAGC TG in das L1 ORF des Humanen Papillomavirus Typ 16 (HPV 16) eingefügt ist.

20. Virusähnliche Partikel nach einem der Ansprüche 13 bis 15, **dadurch gekennzeichnet, daß** es sich bei der mindestens einen Deletion im C-terminalen Bereich im L1- und/oder L2-Protein um eine homologe Aminosäuresequenz des humanen Papillomavirus 16 oder anderer Papillomaviren handelt.

21. Virusähnliche Partikel nach einem der Ansprüche 12 bis 20, **dadurch gekennzeichnet, daß** die Deletion des L1- und/oder L2-Proteins das nukleäre Lokalisationssignal (NLS) umfaßt.

22. Virusähnliche Partikel nach einem der Ansprüche 12 bis 21, **dadurch gekennzeichnet, daß** die Partikelproduktion aus den L1-Proteinen oder L1- und L2-Proteinen im Zytoplasma erfolgt.

23. Virusähnliche Partikel nach einem der Ansprüche 12 bis 22, **dadurch gekennzeichnet, daß** Partikel in den Überstand sezerniert werden.

24. Virusähnliche Partikel nach Anspruch 23, **dadurch gekennzeichnet, daß** ca. 5 bis 10 % der Partikel in den Überstand sezerniert werden.

25. Virusähnliche Partikel nach einem der Ansprüche 12 bis 24, **dadurch gekennzeichnet, daß** die Expression von L1-Proteinen oder L1- und L2-Proteinen in E. coli erfolgt.

26. Virusähnliche Partikel nach Anspruch 25, **dadurch gekennzeichnet, daß** an der C-terminalen Deletion im L1 Protein 6 Histidine eingefügt wurden.

27. Virusähnliche Partikel nach einem der Ansprüche 25 bis 26, **dadurch gekennzeichnet, daß** die Herstellung von VLPs nach Expression von L1-Proteinen oder L1-und L2-Proteinen in E. coli erfolgt.

28. Virusähnliche Partikel nach einem der Ansprüche 12 bis 27, **dadurch gekennzeichnet, daß** es sich bei den weiteren deletierten Bereichen im L1-Protein um die Aminosäuresequenzen 311-351, 331-371, 391-431 des Bovinen Papillomavirus Typ 1 handelt.

29. Virusähnliche Partikel nach Anspruch 27, **dadurch gekennzeichnet, daß** es sich bei den weiteren deletierten Bereichen im L1-Protein um die Aminosäuresequenz 306-315 des humanen Papillomavirus 16 handelt.

30. Virusähnliche Partikel nach einem der Ansprüche 12 bis 29, **dadurch gekennzeichnet, daß** der deletierte Bereich von L1- und/oder L2-Proteinen durch andere Proteine oder Proteinfragmente ersetzt ist, um ein Fusionsprotein zu erhalten.

31. Virusähnliche Partikel nach Anspruch 30, **dadurch gekennzeichnet, daß** der Anteil an L1- bzw. L2-Protein ca. 50 bis 99 %, bevorzugt ca. 60 bis 90 %, besonders bevorzugt ca. 80 % des Fusionsproteins beträgt.

32. Virusähnliche Partikel nach Anspruch 30 und 31, **dadurch gekennzeichnet, daß** der deletierte Bereich im L1- oder L2-Protein durch andere Proteine von Papillomaviren und/oder Proteinen anderen Ursprungs ersetzt ist, wodurch virusähnliche Hybridpartikel herstellbar sind.

33. Virusähnliche Partikel nach einem der Ansprüche 12 bis 32, **dadurch gekennzeichnet, daß** die Bildung der virusähnlichen Partikel aus einem L1-Protein, einem L1-Fusionsprotein, einem L1-Protein und L2-Protein, einem L1-Fusionsprotein und L2-Protein, einem L1-Protein und einem L2-Fusionsprotein oder einem L1-Fusionsprotein und einem L2-Fusionsprotein erfolgt.

34. Virusähnliche Partikel nach einem der Ansprüche 12 bis 33, **dadurch gekennzeichnet, daß** es sich bei mindestens einem der deletierten Bereiche im L1-und/oder L2-Protein eines Papillomavirus um N-terminale Aminosäuresequenzen handelt.

35. Virusähnliche Partikel nach einem der Ansprüche 12 bis 34, **dadurch gekennzeichnet, daß** es sich bei mindestens einem der deletierten Bereiche im L1-und/oder L2-Protein eines Papillomavirus um Aminosäuresequenzen im mittleren Bereich des Proteins handelt.

36. Proteine, insbesondere zur Bildung von papillomavirusähnlichen Hybridpartikeln gemäß einem der Ansprüche 12 bis 35, **dadurch gekennzeichnet, daß** ein oder mehrere Abschnitte des L1- und/oder L2-Proteins deletiert sind.

37. Proteine nach Anspruch 36, **dadurch gekennzeichnet, daß** es sich bei mindestens einem der deletierten Bereiche im L1- und/oder L2-Protein um eine Deletion einer C-terminalen Aminosäuresequenz handelt.

38. Fusionsproteine, insbesondere zur Bildung von papillomavirusähnlichen Hybridpartikeln nach einem der Ansprüche 12 bis 37, **dadurch gekennzeichnet, daß** die Fusionsproteine deletierte L1- und/oder L2-Proteine von verschiedenen Papillomaviren und andere Proteine oder Fragmente von Papillomaviren oder anderer Herkunft enthalten.

39. Fusionsprotein nach Anspruch 38, **dadurch gekennzeichnet, daß** die anderen Proteine oder Fragmente frühe Papillomavirus-Proteine oder Fragmente davon sind.

40. Fusionsprotein nach Anspruch 38 oder 39, **dadurch gekennzeichnet, daß** es sich um die frühen Proteine E1, E2, E4, E5, E6, E7 oder um Fragmente davon handelt.

41. Fusionsprotein nach einem der Ansprüche 38 bis 40, **dadurch gekennzeichnet, daß** es sich um deletierte L1-und/oder L2-Proteine von verschiedenen HPV-Typen wie HPV 6, 11, 16, 18, 31, 33, 35, 45 handelt.

42. Verfahren zur Expression der Fusionsproteine gemäß einem der Ansprüche 8 bis 11, **dadurch gekennzeichnet, daß** die Expression der Proteine und Fusionsproteine in viralen oder in eukaryotischen Vektoren durchgeführt wird.

43. Verfahren zur Expression der Proteine und/oder Fusionsproteine und Produktion von papillomavirusähnlichen Partikeln gemäß einem der Ansprüche 12 bis 35, **dadurch gekennzeichnet, daß** die Expression der Proteine und Fusionsproteine in viralen, eukaryotischen oder prokaryotischen Vektoren durchgeführt wird.

44. Verfahren nach einem der Ansprüche 42 oder 43, **dadurch gekennzeichnet, daß** die Proteine oder Fusionsproteine durch Expression in Vaccinia-Rekombinanten hergestellt werden.

45. Verfahren nach einem der Ansprüche 42 oder 43, **dadurch gekennzeichnet, daß** die Expression der Proteine und Fusionsproteine in Baculoviren oder in Hefen durchgeführt wird.

46. Verfahren nach Anspruch 43, **dadurch gekennzeichnet, daß** die Expression der Proteine und Fusionsproteine in E. coli durchgeführt wird.

47. Verfahren nach Anspruch 46, **dadurch gekennzeichnet, daß** die Reinigung, insbesondere der L1-Proteine, durch Ni-Affinitätschromatographie und Renaturierung, bevorzugt in 150 mM NaCl, 1 mM CaCl₂, 0,01 % Triton-X 100, 10 mM Hepes pH 7,4, erfolgt.

48. Verfahren nach Anspruch 47, **dadurch gekennzeichnet, daß** die Proteine zum Zeitpunkt der Reinigung in Denaturierungspuffer vorliegen.

49. Verfahren nach Anspruch 48, **dadurch gekennzeichnet, daß** es sich bei dem Denaturierungspuffer um 6 M Guanidinhydrochlorid handelt.

50. Verfahren nach einem der Ansprüche 43 bis 49, **dadurch gekennzeichnet, daß** das Assembly der VLPs nach Dialyse erfolgt.

51. Verfahren nach Anspruch 50, **dadurch gekennzeichnet, daß** die Dialyse gegen 150 mM NaCl, 25 mM Ca²⁺, 10% DMSO, 0,1% Triton-X 100 und 10 mM Tris Essigsäure bei pH = 5,0 erfolgt.

52. Verfahren nach einem der Ansprüche 43 bis 51, **dadurch gekennzeichnet, daß** die Partikelproduktion im Zytoplasma erfolgt.

53. Verfahren nach einem der Ansprüche 43 bis 52, **dadurch gekennzeichnet, daß** Partikel in den Überstand sezerniert werden.

54. Verfahren nach Anspruch 53, **dadurch gekennzeichnet, daß** ungefähr 5 bis 10% der Partikel in den Überstand sezerniert werden.

55. Verwendung der Proteine und/oder Fusionsproteine gemäß einem der Ansprüche 8 bis 11 oder 36 bis 41 zur Herstellung eines Impfstoffs zur prophylaktischen und/oder therapeutischen Impfung, bevorzugt nach Zugabe weiterer Komponenten.

56. Verwendung der virusähnlichen Hybridpartikel gemäß einem der Ansprüche 1 bis 7 oder 12 bis 35 zur Herstellung eines Impfstoffs zur prophylaktischen und/oder therapeutischen Impfung, bevorzugt nach Zugabe weiterer Komponenten.
